# EUROPÄISCHE PATENTSCHRIFT

(11) **EP 1 202 958 B1**
(45) Veröffentlichungstag und Bekanntmachung des Hinweises auf die Patenterteilung: **22.10.2003**
(21) Anmeldenummer: 00956404.8
(22) Anmeldetag: 07.08.2000
(51) Int. Cl.: C07C 261/02, C08G 73/06

(54) **DICYANATOCHALKONE UND VERFAHREN ZU DEREN HERSTELLUNG**
DICYANATOCHALCONES AND METHOD FOR THE PRODUCTION THEREOF
DICYANATOCHALCONES ET LEUR PROCEDE DE PRODUCTION

(30) Priorität: 10.08.1999 EP 99115759; 03.09.1999 US 152250 P
(43) Veröffentlichungstag der Anmeldung: 08.05.2002
(73) Patentinhaber: Lonza AG, 4052 Basel (CH)
(72) Erfinder: DAS, Sajal, Bedminster, NJ 07921 (US); DAUM, Ulrich, CH-4114 Hofstetten (CH); FENGLER-VEITH, Marion, CH-8038 Zürich (CH); WILLA, Pascal, CH-3945 Getwing (CH)
(86) Internationale Anmeldenummer: EP0007629
(87) Internationale Veröffentlichungsnummer: WO01010824

(56) Entgegenhaltungen:
- WO-A-90/01514
- PATENT ABSTRACTS OF JAPAN vol. 1999, no. 04, 30. April 1999 (1999-04-30) & JP 11 021503 A (HITACHI CHEM CO LTD), 26. Januar 1999 (1999-01-26)
- PATENT ABSTRACTS OF JAPAN vol. 1999, no. 04, 30. April 1999 (1999-04-30) & JP 11 021504 A (HITACHI CHEM CO LTD), 26. Januar 1999 (1999-01-26)

## Beschreibung

Die Erfindung betrifft Dicyanatochalkone der allgemeinen Formel 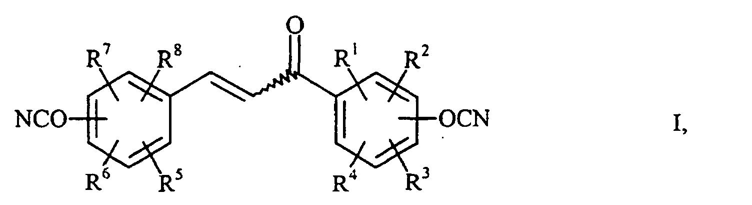 worin R¹ bis R⁸ unabhängig voneinander Wasserstoff, C₁₋₄-Alkyl, C₁₋₄-Alkoxy oder Halogen bedeuten.

Unter C₁₋₄-Alkyl sind hier und im folgenden alle linearen oder verzweigten primären, sekundären oder tertiären Alkylgruppen mit 1 bis 4 Kohlenstoffatomen zu verstehen, also Methyl, Ethyl, Propyl, Isopropyl, Butyl, Isobutyl, *sec*-Butyl und *tert*-Butyl.

Entsprechend sind unter C₁₋₄-Alkoxy die aus C₁₋₄-Alkyl und Sauerstoff zusammengesetzten Gruppen zu verstehen.

Unter Halogen sind Fluor, Chlor, Brom und Iod zu verstehen, insbesondere Chlor und Brom.

Difunktionelle aromatische Cyanate wie beispielsweise das aus Bisphenol A abgeleitete 2,2-Bis(4-cyanatophenyl)propan sind Ausgangsmaterialien für Polytriazinharze, die wegen ihrer hohen Wärmebeständigkeit und günstigen mechanischen und elektrischen Eigenschaften immer mehr Anwendungen finden. Trotz dieser Vorteile sind deren Eigenschaften noch nicht in jeder Hinsicht zufriedenstellend. So sind beispielsweise Flexibilität, Schlagzähigkeit, Bruchdehnung und Weiterreissfestigkeit für gewisse Anwendungen ungenügend, ausserdem kann die Exothermie bei der Aushärtung zu Problemen führen.

Aufgabe der vorliegenden Erfindung war daher, neue aromatische Cyanate bereitzustellen, die eine geringe Exothermie bei der Cyclotrimerisierung zu Polytriazinen aufweisen und Polytriazine mit verbesserten mechanischen Eigenschaften liefern.

Erfindungsgemäss wird diese Aufgabe durch die Dicyanatochalkone nach Patentanspruch 1 gelöst.

Es wurde gefunden, dass Dicyanatochalkone der allgemeinen Formel 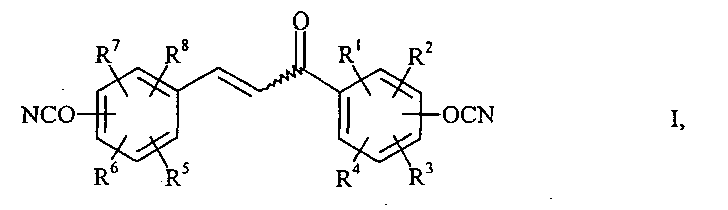 worin R¹ bis R⁸ unabhängig voneinander Wasserstoff, C₁₋₄-Alkyl, C₁₋₄-Alkoxy oder Halogen bedeuten, nur eine geringe Exothermie bei der Cyclotrimerisierung zu Polytriazinen aufweisen. Weiterhin ergeben sie Polytriazinharze mit hoher Flexibilität, Schlagzähigkeit, Weiterreissfestigkeit und Bruchdchnung sowie niedriger Dielektrizitätskonstante. Im Brandfall bilden sie ausserdem nur wenig Rauchgase von geringer Toxizität. Ausserdem ist die freiwerdende Energie bei der Verbrennung der ausgehärteten Harze und/oder Bauteile äusserst gering ("peak heat release") im Vergleich zu den bisher erhältlichen Cyanatestern oder anderen Harzsystemen.

Besonders bevorzugt ist das 1,3-Bis-(4-cyanatophenyl)-2-propen-1-on der Formel

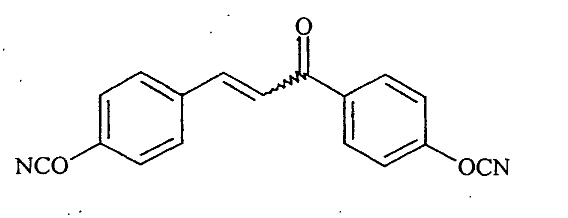

Die erfindungsgemässen Dicyanatochalkone können dadurch hergestellt werden, dass ein Dihydroxychalkon der allgemeinen Formel 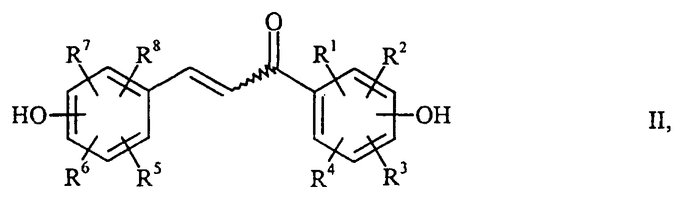 worin R¹ bis R⁸ die oben genannten Bedeutungen haben,
mit Chlorcyan oder Bromcyan und einem tertiären Amin umgesetzt wird.

Die Dihydroxychalkone (II) sind bekannte Verbindungen oder analog zu bekannten Verfahren aus den entsprechenden substituierten Benzaldehyden und Acetophenonen erhältlich.

Die Umsetzung des Dihydroxychalkons (II) wird vorzugsweise mit Chlorcyan durchgeführt.

Als tertiäres Amin ist Triethylamin besonders bevorzugt.

Die folgenden Beispiele verdeutlichen die Herstellung der erfindungsgemässen Verbindungen, ohne dass darin eine Einschränkung zu sehen ist.

### Beispiel 1

### 1,3-Bis-(4-cyanatophenyl)-2-propen-1-on

In einem doppelwandigen Reaktor (250 ml) mit Rührer, Thermometer, Gaseinleitungsrohr und Flüssigkeitsdosierpumpe wurden 10 g (41,5 mmol) 1,3-Bis-(4-hydroxyphenyl)-2-propen-1-on vorgelegt. Nach Zugabe von 140 ml wasserfreiem Aceton wurde die Lösung auf-10 °C abgekühlt. Dann wurden zuerst innerhalb von 10 min 5,63 g (91,5 mmol) Chlorcyan eingeleitet und dann unter heftigem Rühren 8,55 g (84,5 mmol) Triethylamin mit einer Flussrate von ca. 0,4 ml/min zudosiert, wobei die Temperatur des Reaktionsgemischs ständig unter -10 °C gehalten wurde. Dann wurde das Reaktionsgemisch auf 20 °C erwärmt, bei dieser Temperatur 30 min gerührt und wieder auf 0 °C abgekühlt. Das gebildete Triethylammoniumchlorid wurde abfiltriert und mit kaltem Aceton (2×50 ml) gewaschen. Das Filtrat wurde am Rotationsverdampfer (20 mbar, 40 °C Badtemperatur) auf 70 g eingeengt, dann bei 20 °C mit der gleichen Menge Wasser versetzt und schliesslich auf 5 °C abgekühlt. Das auskristallisierte Produkt wurde abfiltriert und im Vakuumtrockenschrank 16 h bei 20 mbar/50 °C getrocknet. Weiteres Produkt wurde durch Eindampfen der Mutterlauge und Umkristallisieren des Rückstands aus wässrigem Aceton erhalten.
Schmp: 172-174 °C
¹H NMR (d₆-DMSO): δ = 7,54-7,65 (AA'XX', 4H); 7,77-8,00 (AB, 2H); 8,09-8,36 (AA'XX', 4H).
¹³C NMR (d₆-DMSO): δ = 107,9; 108,2; 115,9 (2C); 116,1 (2C); 122,9; 131,3 (2C); 131,5 (2C); 133,6; 136,0; 142,5; 153,5; 155,2.
IR (KBr): 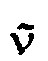 = 3063 (m, CH); 2268, 2235 (vs, OCN); 1717 (w); 1667 (vs); 1615 (m); 1598 (s); 1591 (s); 1500 (s); 1421 (m); 1344 (m); 1328 (m); 1312 (w); 1210 (vs); 1196 (vs); 1167 (s); 1109 (w); 1087 (w); 1032 (m); 1010 (m); 989 (m); 821 (s); 801 (m); 784 (w); 501 (m); 493 (w) cm⁻¹.

### Beispiel 2

### 1,3-Bis-(4-cyanatophenyl)-2-propen-1-on

In einem doppelwandigen Reaktor (250 ml) mit Rührer, Thermometer, Gaseinleitungsrohr und Flüssigkeitsdosierpumpe wurden 10 g (41,5 mmol) 1,3-Bis-(4-hydroxyphenyl)-2-propen-1-on vorgelegt. Nach Zugabe von 110 ml wasserfreiem Aceton wurde die Lösung auf-10 °C abgekühlt. Dann wurden zuerst innerhalb von 10 min 5,63 g (91,5 mmol) Chlorcyan eingeleitet und dann unter heftigem Rühren 8,55 g (84,5 mmol) Triethylamin mit einer Flussrate von ca. 0,4 ml/min zudosiert, wobei die Temperatur des Reaktionsgemischs ständig unter -10 °C gehalten wurde. Dann wurde das Reaktionsgemisch auf 20 °C erwärmt und bei dieser Temperatur 30 min gerührt. Anschliessend wurde die Reaktionsmischung innerhalb von 10 min unter starkem Rühren zu 200 ml Wasser, welches zuvor auf 5 °C gekühlt worden war, gegeben. Die Suspension wurde darauf 30 min gerührt, langsam auf 15 °C erwärmt und filtriert. Der Filterkuchen wurde mit Wasser gewaschen (2×30 ml) und im Vakuumtrockenschrank 20 h bei 20 mbar/50 °C getrocknet. Auf diese Weise wurden 11,03 g (92%) Cyanat mit einem Schmelzpunkt von 171-173°C isoliert.

## Patentansprüche

1. Dicyanatochalkone der allgemeinen Formel 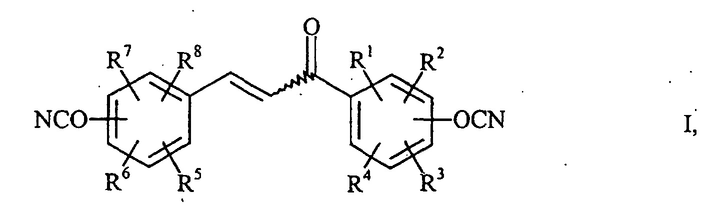 worin R¹ bis R⁸ unabhängig voneinander Wasserstoff, C₁₋₄-Alkyl, C₁₋₄-Alkoxy oder Halogen bedeuten.

2. 1,3-Bis-(4-cyanatophenyl)-2-propen-1-on der Formel

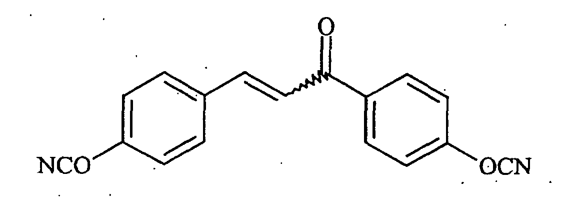

3. Verfahren zur Herstellung von Dicyanatochalkonen der allgemeinen Formel 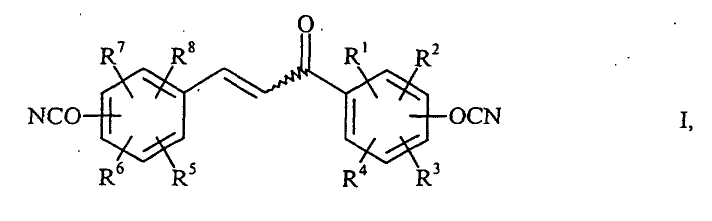 worin R¹ bis R⁸ unabhängig voneinander Wasserstoff, C₁₋₄-Alkyl, C₁₋₄-Alkoxy oder Halogen bedeuten, **dadurch gekennzeichnet, dass** ein Dihydroxychalkon der allgemeinen Formel 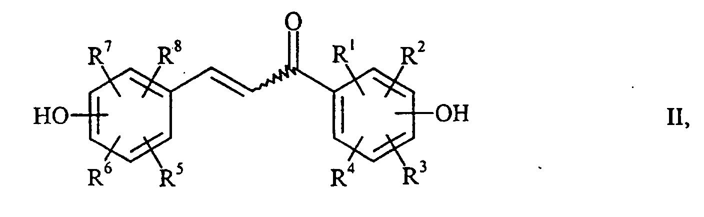 worin R¹ bis R⁸ die oben genannten Bedeutungen haben,
mit Chlorcyan oder Bromcyan und einem tertiären Amin umgesetzt wird.

4. Verfahren nach Anspruch 3, **dadurch gekennzeichnet, dass** die Umsetzung mit Chlorcyan durchgeführt wird.

5. Verfahren nach Anspruch 3 oder 4, **dadurch gekennzeichnet, dass** als tertiäres Amin Triethylamin eingesetzt wird.

## Claims

1. Dicyanatochalcones of the general formula 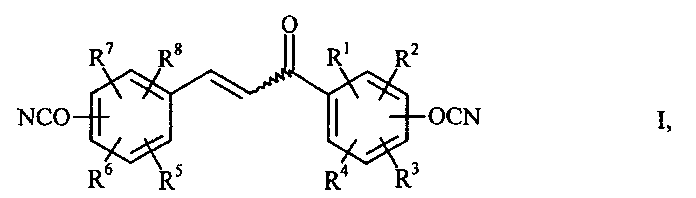 in which R¹ to R⁸ independently of one another are hydrogen, C₁₋₄-alkyl, C₁₋₄-alkoxy or halogen.

2. 1,3-Bis-(4-cyanatophenyl)-2-propen-1-one of the formula

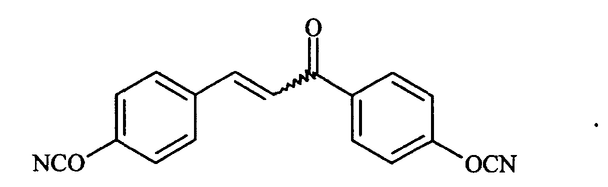

3. Process for preparing dicyanatochalcones of the general formula 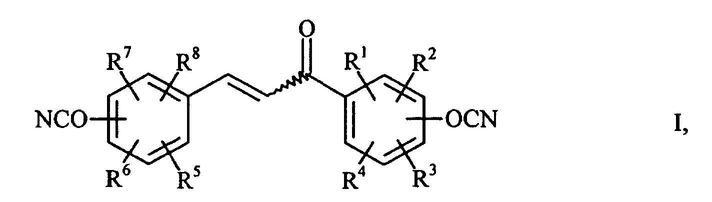 in which R¹ to R⁸ independently of one another are hydrogen, C₁₋₄-alkyl, C₁₋₄-alkoxy or halogen, **characterized in that** a dihydroxychalcone of the general formula 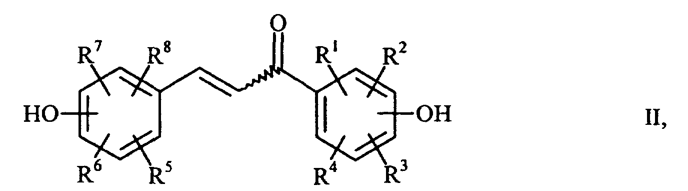 in which R¹ to R⁸ are as defined above is reacted with cyanogen chloride or cyanogen bromide and a tertiary amine.

4. Process according to Claim 3, **characterized in that** the reaction is carried out using cyanogen chloride.

5. Process according to Claim 3 or 4, **characterized in that** the tertiary amine used is triethylamine.

## Revendications

1. Dicyanatochalcones de formule générale 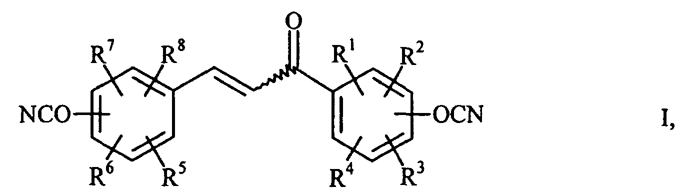 dans laquelle R¹ à R⁸ représentent, indépendamment l'un de l'autre, un atome d'hydrogène ou d'halogène ou un groupe alkyle en C₁₋₄ ou alcoxy en C₁₋₄.

2. 1,3-Bis(4-cyanatophényl)-2-propén-1-one de formule

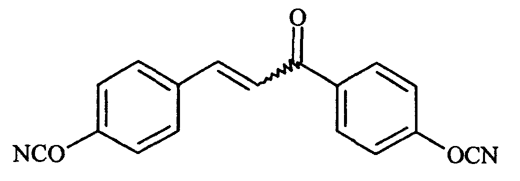

3. Procédé pour la préparation de dicyanatochalcones de formule générale 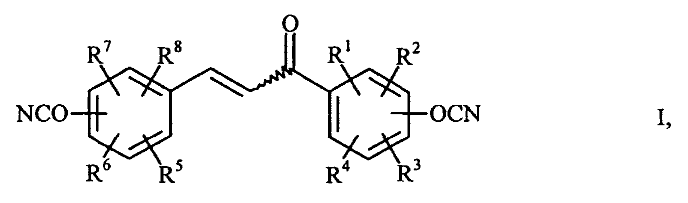 dans laquelle R¹ à R⁸ représentent, indépendamment l'un de l'autre, un atome d'hydrogène ou d'halogène ou un groupe alkyle en C₁₋₄ ou alcoxy en C₁₋₄, **caractérisé en ce qu'**on fait réagir une dihydroxychalcone de formule générale 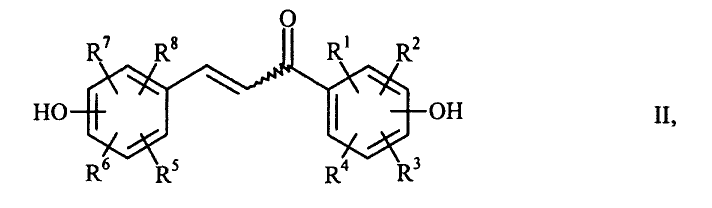 dans laquelle R¹ à R⁸ ont les significations données plus haut, avec du chlorure de cyanogène ou du bromure de cyanogène et un amine tertiaire.

4. Procédé selon la revendication 3, **caractérisé en ce que** la réaction est effectuée avec du chlorure de cyanogène.

5. Procédé selon la revendication 3 ou 4, **caractérisé en ce qu'**on utilise comme amine tertiaire la triéthylamine.
